# FASCICULE DE BREVET EUROPEEN

(11) **EP 0 684 041 B1**
(45) Date de publication et mention de la délivrance du brevet: **09.04.1997**
(21) Numéro de dépôt: 95400886.8
(22) Date de dépôt: 20.04.1995
(51) Int. Cl.: A61K 7/48, A61K 7/06

(54) **Compositions cosmétiques contenant un copolymère bloc linéaire polysiloxane-polyoxyalkylène, un agent de conditionnement insoluble non volatile et un alcool hydrosoluble, et leurs utilisations**
Kosmetische Zusammensetzungen, die ein lineares Polysiloxan-Polyoxyalkylen-Blockcopolymers, ein unlösliches nichtflüchtiges Konditioniermittel, und ein wasserlöslicher Alkohol enthalten, und ihre Verwendungen
Cosmetic compositions containing a linear polysiloxane-polyoxyalkylene block-copolymer, a non volatile insoluble conditioning agent, and a water-soluble alcohol, and their use

(30) Priorité: 26.05.1994 FR 9406395
(43) Date de publication de la demande: 29.11.1995
(73) Titulaire: L'OREAL, 75008 Paris (FR)
(72) Inventeur: Dupuis, Christine, F-75018 Paris (FR)
(74) Mandataire: Tezier Herman, Béatrice

(56) Documents cités:
- EP-A- 0 469 602
- EP-A- 0 492 657
- EP-A- 0 595 683

## Description

La présente invention concerne de nouvelles compositions cosmétiques comprenant dans un milieu aqueux au moins un copolymère bloc linéaire polysiloxane-polyoxyalkylène, au moins un alcool hydrosoluble et au moins un agent de conditionnement insoluble choisi parmi les silicones non volatiles et les huiles ou cires fluorées.

Il est bien connu que des cheveux qui ont été sensibilisés (i.e. abîmés et/ou fragilisés) à des degrés divers sous l'action d'agents atmosphériques ou sous l'action de traitements mécaniques ou chimiques tels que des colorations, des décolorations et/ou des permanentes, sont souvent difficiles à démêler et à coiffer, et manquent de douceur.

L'un des moyens couramment utilisés pour améliorer le démêlage et la douceur de ces cheveux consiste à appliquer sur ces derniers des compositions cosmétiques contenant un agent de conditionnement.

A cet égard, l'utilisation dans des compositions cosmétiques pour cheveux et/ou la peau, et plus particulièrement dans celles qui sont appelées à être appliquées sur des cheveux sensibilisés, d'agents cosmétiques complémentaires de type silicones, dérivés de silicones, huiles ou cires fluorées, qui apportent alors aux cheveux traités une facilité de démêlage et de coiffage, ainsi qu'une douceur et une brillance nettement accrues, est aujourd'hui largement répandue.

Toutefois, l'emploi de tels agents de conditionnement ne va pas sans poser certains problèmes.

Tout d'abord, compte tenu du caractère insoluble de ces agents de conditionnement dans les milieux aqueux , il est nécessaire de les mettre en oeuvre sous une forme dispersée. En outre, les agents de conditionnement insolubles non volatiles, en particulier les silicones de haut poids moléculaires, les gommes ou les résines de silicone, présentent une viscosité importante et leurs mises en oeuvre dans des compositions aqueuses est donc particulièrement difficile.

On sait émulsionner les silicones en présence de tensioactifs, mais de nombreux paramètres (nature et concentrations du ou des tensioactifs, système d'agitation, température, ...) sont à prendre en considération pour obtenir un bon résultat. Ces préparations nécessitent en outre la mise en oeuvre d'une quantité d'énergie importante (agitation et/ou chauffage).

On a aussi proposé d'introduire ces silicones dans des silicones volatiles ; le mélange présente alors une viscosité plus faible mais la dispersion dans les milieux aqueux ne se fait pas. On a également préconisé d'utiliser des silicones présentant des propriétés tensioactives tels que les diméthicones copolyol (dénomination CTFA 5ème édition 1993) ou leurs mélanges avec des silicones volatiles pour émulsionner les silicones non volatiles, mais les résultats ne sont pas satisfaisants. Les dispersions sont grossières.

D'une manière générale, on notera en outre que les émulsionnants utilisés à ce jour conduisent à des compositions dont les propriétés cosmétiques peuvent apparaître comme encore insuffisantes.

La demande EP-A-469602 décrit des compositions cosmétiques comprenant une poudre hydrophobe et un composé organique perfluoré liquide. La demande EP 595 683 décrit des émulsions eau dans huile (E/H) comprenant un tensioactif siliconé et un fluorohydrocarbure.

La demande EP-A492657 décrit des compositions cosmétiques contenant des copolymères bloc linéaire polysiloxane-polyoxyalkylène de formule (I).

La présente invention a ainsi pour but de proposer un nouveau moyen permettant de faciliter la mise en oeuvre d'agents de conditionnement choisis parmi les silicones non volatiles insolubles, les huiles ou les cires fluorées, dans les milieux aqueux, c'est à dire d'obtenir des dispersions fines de ces agents de conditionnement dans ces milieux.

L'invention a également pour but de proposer des compositions cosmétiques présentant des propriétés cosmétiques améliorées, en particulier au niveau de leur pouvoir démêlant et de la douceur.

Or, la demanderesse a maintenant découvert que le mélange d'un copolymère bloc linéaire polysiloxane-polyoxyalkylène selon la revendication 1 et d'au moins un alcool hydrosoluble permettait de mettre facilement en dispersion dans les milieux aqueux, les agent de conditionnement insolubles choisi parmi les silicones non volatiles et les huiles ou les cires fluorées. Les dispersions obtenues sont fines et présentent de bonnes propriétés cosmétiques. Elles sont obtenues par simple agitation, l'utilisation de matériel sophistiqué n'étant pas nécessaire.

L'invention a donc pour objet une composition cosmétique comprenant, dans un milieu aqueux cosmétiquement acceptable, au moins un agent de conditionnement insoluble choisi parmi les silicones non volatiles et les huiles ou les cires fluorées, au moins un alcool hydrosoluble et au moins un copolymère bloc linéaire polysiloxane-polyoxyalkylène selon la revendication 1.

Un autre objet de l'invention est constitué par l'utilisation de ces compositions pour le maintien et/ou la fixation de la coiffure, le traitement, le soin ou le lavage des cheveux, de la peau ou de toute autre matière kératinique, ainsi que par l'utilisation d'un mélange copolymère bloc linéaire polysiloxane-polyoxyalkylène selon la revendication 1/alcool hydrosoluble comme agent émulsionnant pour un selon la revendication 1/alcool hydrosoluble comme agent émulsionnant pour un agent de conditionnement insoluble choisi parmi les silicones non volatiles et les huiles ou les cires fluorées dans une composition cosmétique aqueuse contenant un tel agent de conditionnement.

Les copolymères blocs linéaires polysiloxane-polyoxyalkylène utilisés dans le cadre de la présente invention ont la formule générale suivante :

([ Y (R₂SiO)ₐ R'₂SiYO][( CₙH₂ₙO)_{b}])_{c}

dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5,
- b est un nombre entier supérieur ou égal à 4,
- c est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 95% environ en poids du copolymère bloc,
- le poids moléculaire moyen du copolymère bloc étant d'au moins 3.000.

R et R' sont préférentiellement choisis parmi le groupe comprenant les radicaux alkyles comme par exemple les radicaux méthyle, éthyle, propyle, butyle, pentyle, hexyle, octyle, décyle, dodécyle, les radicaux aryles comme par exemple phényle, naphtyle, les radicaux aralkyles comme par exemple benzyle, phényléthyle, les radicaux tolyle, xylyle et cyclohexyle.

Y est de préférence -R''-, -R''-CO-, -R''-NHCO-, -R''-NH-CO-NH-R'''-NHCO,--R''-OCONH-R'''-NHCO-, où R'' est un groupe alkylène divalent comme par exemple l'éthylène, le propylène ou le butylène et R''' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical C₄H₈.

La préparation des copolymères blocs mis en oeuvre dans le cadre de la présente invention est décrite dans la demande européenne EP 0 492 657 A1, dont l'enseignement est inclus à titre de référence dans la présente description.

Les copolymères blocs linéaires polysiloxane-polyoxyalkylène préférés selon l'invention sont choisis parmi ceux de formule:

[C₄H₈O(CₙH₂ₙO)_{b}(CₘH₂ₘO)_{d}-C₄H₈-SiMe₂O(SiMe₂O)ₚSiMe₂]_{c}

où Me représente méthyle , n et m sont des entiers allant de 2 à 4 , p est un entier supérieur ou égal à 4, b et d sont des entiers supérieurs ou égaux à 0, b + d est supérieur ou égal à 4 et c est un nombre supérieur ou égal à 4.

Parmi ces copolymères, on utilise plus particulièrement ceux ayant un motif récurrent de formule :

[-(SiMe₂O)ₓSiMe₂-C₄H₈O-(C₂H₄O)y-(C₃H₆O)_{z}-C₄H₈-]

où x est un nombre compris entre 5 et 15 (bornes incluses), y est un nombre compris entre 15 et 30 (bornes incluses); et z est un nombre compris entre 20 et 40 (bornes incluses).

Selon un mode de réalisation particulier de l'invention le copolymère bloc est choisi parmi les copolymères suivants :
[[(CH₃)₂SiO]₄₁(CH₃)₂SiCH₂CH(CH₃)CH₂-O(C₂H₄O)₁₈-(C₃H₆O)₃₃CH₂CH(CH₃)CH₂]_{16.1}
[[(CH₃)₂SiO]₃₁(CH₃)₂SiCH₂CH(CH₃)CH₂-O(C₂H₄O)₂₀-(C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈-(C₃H₆O)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}

Le copolymère bloc linéaire est utilisé de préférence en une quantité comprise entre 0,05 et 15% en poids du poids total de la composition. Encore plus préférentiellement, cette quantité est comprise entre 0,1 et 10% en poids.

Les silicones insolubles non volatiles utilisables dans le cadre de la présente invention peuvent être choisies parmi toutes celles déjà connues en soi comme améliorant les propriétés cosmétiques des cheveux traités par des compositions cosmétiques, à savoir notamment celles décrites dans les demandes de brevets EP-A- 0 181773 et EP-A- 0 473508, dont les enseignements sont ici inclus à titre de référence. Il est bien entendu possible de mettre en oeuvre des mélanges de silicones.

Ainsi, selon la présente invention, il est possible d'utiliser toute silicone non volatiles connue en soi, qu'il s'agisse d'une huile, d'une résine ou bien encore d'une gomme de silicone. Les silicones sont des polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalcylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifées"). D'une manière générale, les silicones utilisables dans le cadre de la présente invention sont celles qui sont notamment décrites dans "Encyclopedia of Chemical Technology, Kirk-Othmer, Third Edition, 1982, volume 20, pp. 922 et suivantes" et dans "Chemistry and Technology of Silicones, Walter NOLL, Academic Press Inc, San Diego California, 1968". Le poids moléculaire moyen des silicones utilisables selon l'invention peut varier entre 100 et plusieurs millions, de préférence entre 1000 et 1 000 000. Selon la présente invention, on peut bien entendu soit utiliser une seule et même silicone soit mettre en oeuvre plusieurs silicones différentes.

A titre d'exemples de silicones utilisables dans les compositions selon l'invention, on peut notamment citer les polydialkylsiloxanes, les polyalkylarylsiloxane, les polydiaryldialkylsiloxanes et d'une manière encore plus générale tous les organopolysiloxanes décrits dans la demande de brevet publiée sous le numéro WO 93/05762 et dont l'enseignement est, à cet égard, totalement inclus dans la présente demande à titre de référence.

Selon un mode de réalisation particulièrement préféré de la présente invention, les silicones utilisées sont choisies parmi les diorganopolysiloxanes (huiles, gommes ou résines), de préférence les polydialkylsiloxanes ou les polyalkylarylsiloxanes, et encore plus préférentiellement les polydiméthylsiloxanes éventuellement modifiés.

Les gommes de silicone sont particulièrement préférées et en particulier celles de polydialkylsiloxane ou de polyalkylarylsiloxane. Elles sont utilisées seules ou en mélange dans un solvant choisi par exemple parmi les silicones volatiles, les huiles polydiméthylsiloxane ou polyphénylméthylsiloxane, les isoparaffines, le pentane, le dodécane ou leurs mélanges.
On préfère également les silicones à groupements aminés substitués ou non.

Les huiles ou cires fluorées sont par exemple les perfluoropolyéthers décrits notamment dans la demande de brevet EP-A-486135 et les huiles fluorohydrocarbonées décrites notamment dans la demande de brevet WO 93/11103. L'enseignement de ces deux demandes est totalement inclus dans la présente demande à titre de référence.

Le terme d'huiles fluorohydrocarbonées désigne des composés dont la structure chimique comporte un squelette carboné dont certains atomes d'hydrogène ont été substitués par des atomes de fluor.

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Le ou les agents de conditionnement sont généralement présents dans les compositions conformes à l'invention dans des proportions généralement comprises entre 0,01 à 15 % en poids, de préférence de 0,1 à 10 % en poids, par rapport au poids total de la composition.

Selon l'invention, l'alcool hydrosoluble peut être choisi parmi les alcools en C₁-C₄ tel que l'éthanol, l'éthylèneglycol, le propylène glycol, le butylène glycol, l'isopropanol, les éthers de glycol tel que les alkyl(C₁-C₄)éthers de mono, di ou tripropylène glycol, mono , di ou triéthylène glycol, le dipropylène glycol, le diéthylène glycol et leurs mélanges.

Les alcools hydrosolubles sont généralement présents dans les compositions conformes à l'invention dans des proportions généralement comprises entre 0,5 et 80 % en poids, de préférence de 3 à 40 % en poids et encore plus particulièrement de 5 à 20% en poids, par rapport au poids total de la composition.

La composition de l'invention peut également contenir au moins un additif choisi parmi les épaississants, les esters d'acides gras, les esters d'acides gras et de glycérol, les silicones volatiles, les silicones solubles , les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Les compositions selon l'invention peuvent se présenter sous forme de gel, de lait, de crème, de lotion plus ou moins épaissie ou de mousse.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage, des compositions de coloration, de décoloration, de permanente ou de défrisage des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane et leurs mélanges, éventuellement avec au moins un hydrocarbure chloré et/ou fluoré. On peut également utiliser en tant qu'agent propulseur le gaz carbonique, le protoxyde d'azote, le diméthyléther, l'azote ou l'air comprimé.

L'invention a encore pour objet un procédé de traitement cosmétique de la peau ou des fibres kératiniques telles que les cheveux consistant à appliquer sur ceux-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits.

### EXEMPLE 1

On a préparé des compositions contenant :
- 1 % de silicone non volatile et insoluble
- 1 % d'un composé émulsionnant A
- 42,5 % d'éthanol absolu
- 48 % d'eau
et on a comparé la qualité des dispersions obtenues. Les résultats sont rassemblés dans le tableau ci-dessous.

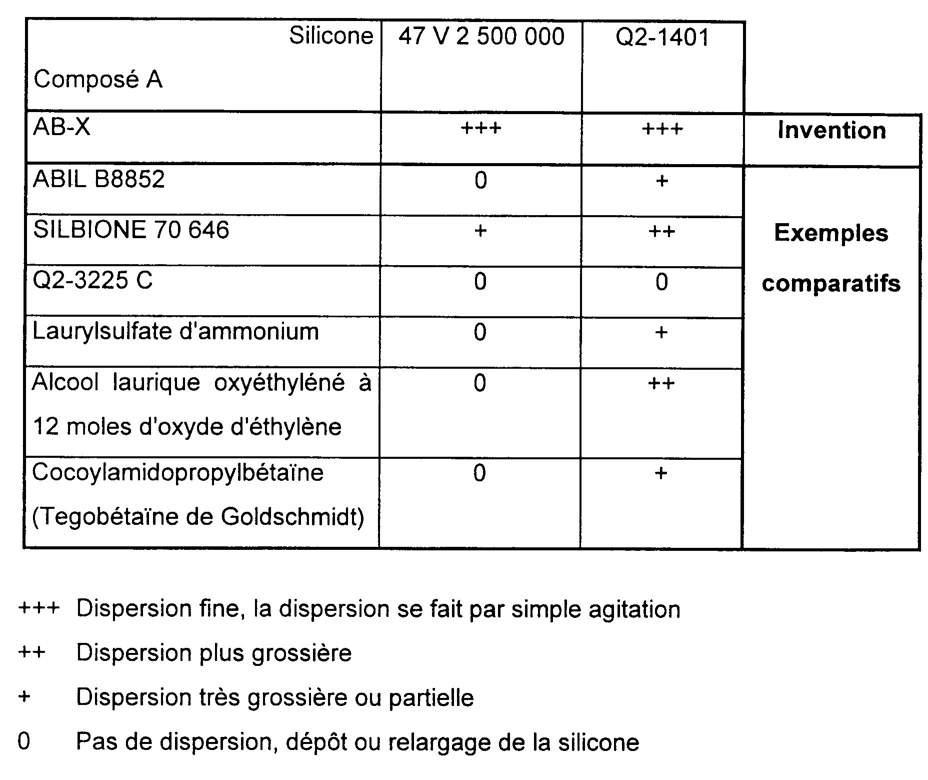

Seules les dispersions avec l'AB-X sont fines et préparées facilement.

On a par ailleurs comparé les propriétés cosmétiques de la composition (I) contenant AB-X + Q2-1401 par rapport à celle (II) contenant SILBIONE 70646 + Q2-1401.

On a appliqué 0,75 g de composition à tester sur 5 g de cheveux humides, puis on a séché les cheveux.
Un panel de 5 juges a ensuite évalué la douceur et le démêlage des mèches de cheveux.

Les 5 juges ont trouvé à l'unanimité que la mèche traitée avec la composition (I) de l'invention était plus douce et que son démêlage était plus facile. Les produits utilisés étaient les suivants :
AB-X =
- Copolymère bloc de formule:

[[(CH₃)₂SiO]ₓ(CH₃)₂SiCH₂CH(CH₃)CH₂O(C₂H₄O)_{y}-(C₃H₆O)_{z}CH₂CH(CH₃)CH₂]ₙ

avec x =9 à 11, y=18 à 25, z=28 à 35 et n est tel que la viscosité d'une solution de polymère à 10 % dans l'éthanol soit d'environ 50 mPa.s.
ABIL B8852 (GOLDSCHMIDT) = Diméthicone copolyol
SILBIONE 70 646 (RHONE POULENC) = Diméthicone copolyol
Q2-3225 C (DOW CORNING) = Diméthicone copolyol en solution à 10% dans des silicones cycliques volatiles (cyclométhicone)
47 V 2 500 000 (RHONE POULENC) = Polydiméthylsiloxane de viscosité 2 500 000 cSt.
Q2-1401 (DOW CORNING) = Mélange (13/87) d'une gomme de diméthiconol et de cyclométhicone

### EXEMPLE 2

On a préparé des compositions contenant :
- 1,5 % de silicone non volatile et insoluble
- 0,5 % d'un composé émulsionnant A
- 42,5 % d'éthanol absolu
- 48 % d'eau
et on a comparé la qualité des dispersions obtenues. Les résultats sont rassemblés dans le tableau ci-dessous.

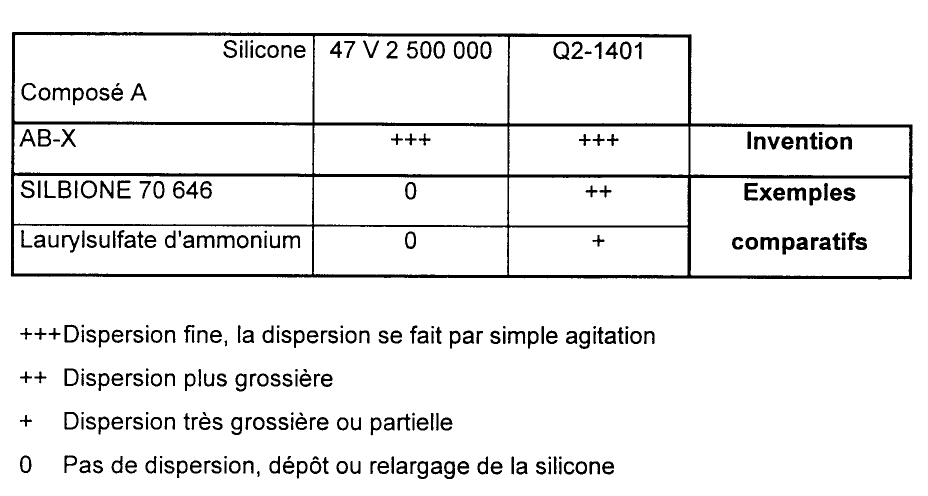

Seules les dispersions avec l'AB-X sont fines et préparés facilement.

### EXEMPLE 3

On a préparé des compositions contenant :
- 1 % de silicone non volatile et insoluble
- 1 % d'un composé émulsionnant A
- 17,6 % d'éthanol absolu
- 80,4 % d'eau
et on a comparé la qualité des dispersions obtenues. Les résultats sont rassemblés dans le tableau ci-dessous.

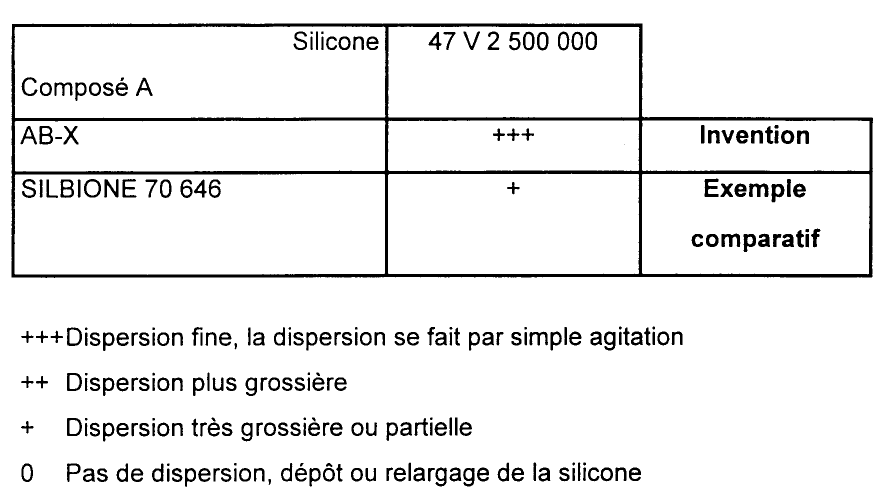

### EXEMPLE 4

On a préparé une composition contenant :

| | |
|---|---|
| - Perfluoroalkyl polyéther (viscosité 60 cSt.) (KRYTOX 1506 de DUPONT) | 1 g |
| - AB-X | 1 g |
| - Ethanol absolu | 42,5 g |
| - Eau qsp | 100 g |

La dispersion est fine et préparée facilement.

Les exemples 5 à 10 donnés ci-après illustrent d'autres formulations concrètes conformes à l'invention.

### EXEMPLE 5

On a préparé un gel de coiffage et de soin capillaire de composition suivante:

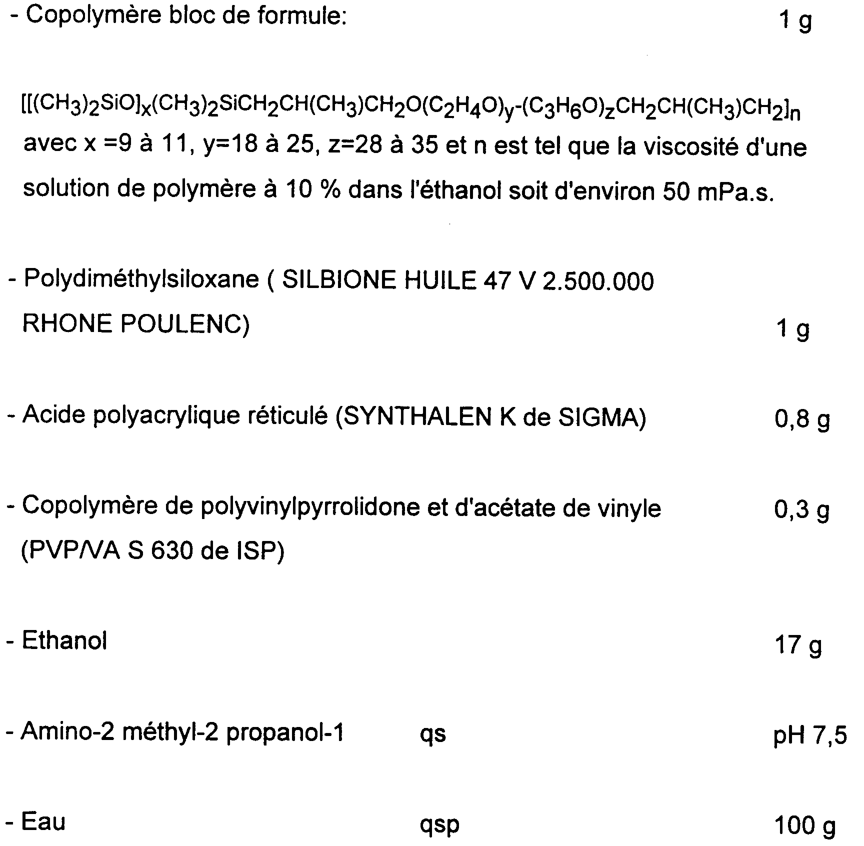

### EXEMPLE 6

On a préparé un gel de soin capillaire de composition suivante:

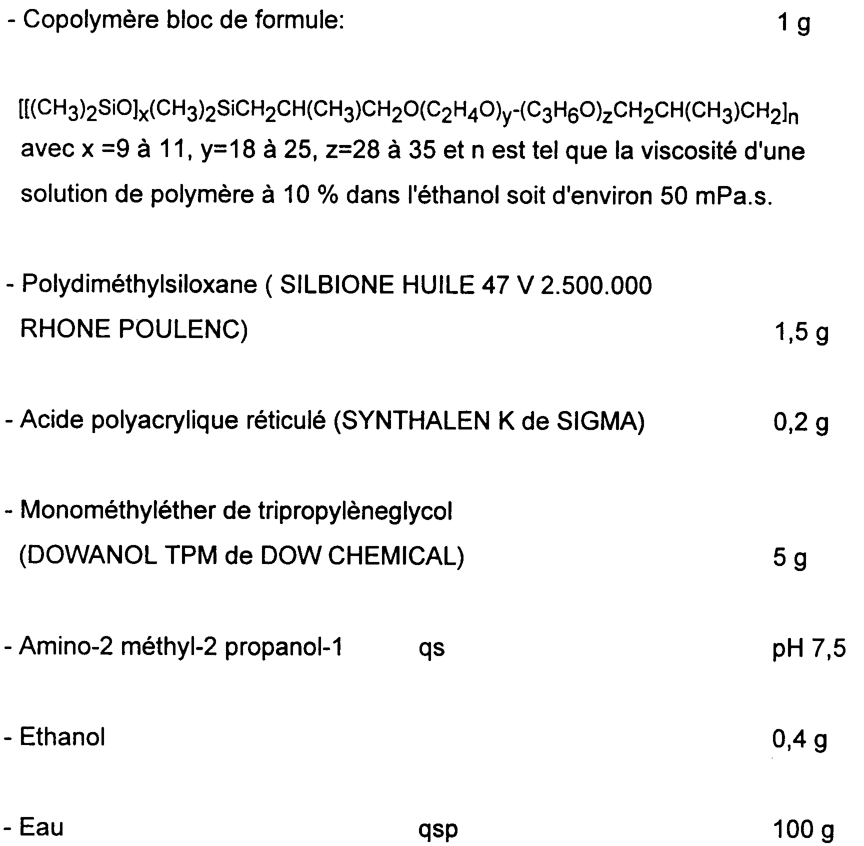

### EXEMPLE 7

On a préparé un gel de soin capillaire de composition suivante:

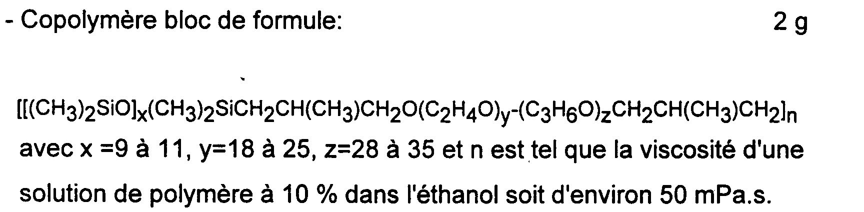

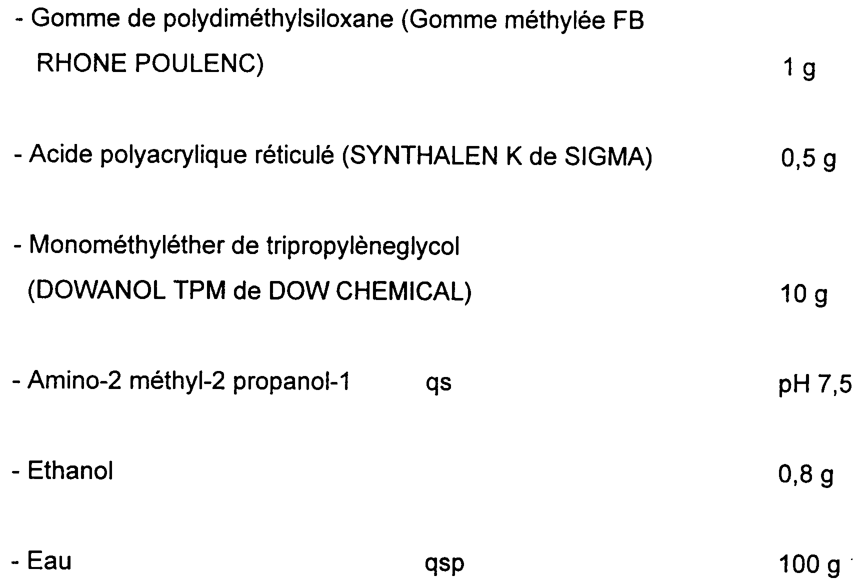

### EXEMPLE 8

On a préparé un spray de soin capillaire conditionné en flacon pompe de composition suivante:

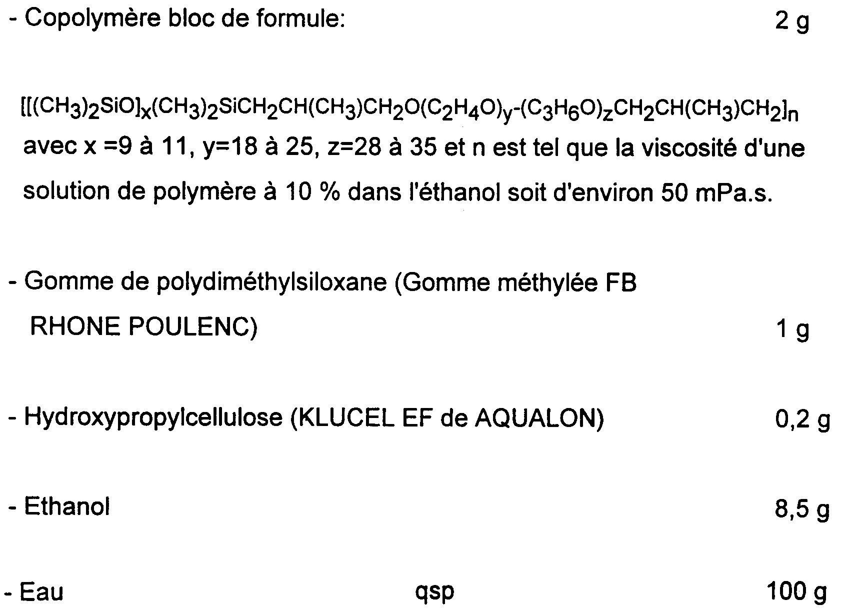

### EXEMPLE 9

On a préparé une lotion de soin capillaire de composition suivante:

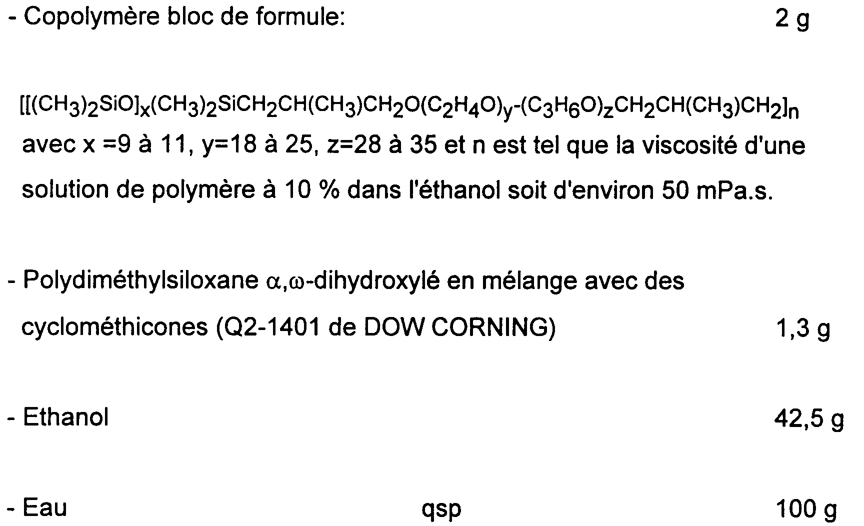

### EXEMPLE 10

On a préparé un gel de soin capillaire de composition suivante:

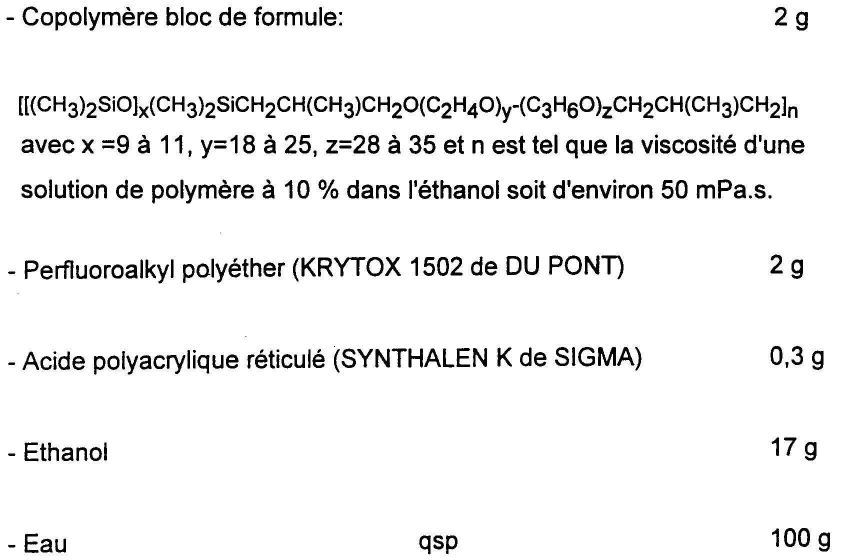

## Revendications

1. Composition cosmétique, caractérisée par le fait qu'elle comprend, dans un milieu aqueux cosmétiquement acceptable, au moins un agent de conditionnement insoluble choisi parmi les silicones non volatiles et les huiles ou cires fluorées, au moins un alcool hydrosoluble et au moins un copolymère bloc linéaire polysiloxane-polyoxyalkylène
répondant à la formule générale:
([ Y (R₂SiO)ₐ R'₂SiYO][( CₙH₂ₙO)_{b}])_{c} (I)
dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5,
- b est un nombre entier supérieur ou égal à 4,
- c est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 95% environ en poids du copolymère bloc,
- le poids moléculaire moyen du copolymère bloc étant d'au moins 3000.

2. Composition selon la revendication 1, caractérisée en ce que R et R' sont choisis dans le groupe constitué par les radicaux méthyle, éthyle, propyle, butyle, pentyle, hexyle, octyle, décyle, dodécyle, phényle, naphtyle, benzyle, phényléthyle, tolyle, xylyle et cyclohexyle.

3. Composition selon l'une quelconque des revendications 1 ou 2, caractérisée en ce que Y est choisi dans le groupe constitué par -R''-, -R''-CO-, -R''-NHCO-, R''-NH-CONH-R'''NHCO-, -R''-OCONH-R'''-NHCO-, où R'' représente un radical éthylène, propylène ou butylène et R''' représente un groupe -C₆H₄-, -C₆H₄-C₆H₄-, -C₆H₄-CH₂-C₆H₄-, ou -C₆H₄-C(CH₃)₂-C₆H₄-.

4. Composition selon l'une quelconque des revendications 1 à 3, caractérisée par le fait que les copolymères blocs linéaires polysiloxane-polyoxyalkylène sont choisis parmi ceux de formule:
[C₄H₈ O(CₙH₂ₙO)_{b} (CₘH₂ₘO)_{d}-C₄H₈-SiMe₂O (SiMe₂O)ₚSiMe₂]_{c}
où Me représente méthyle , n et m sont des entiers allant de 2 à 4 , p est un entier supérieur ou égal à 4, b et d sont des entiers supérieurs ou égaux à 0, b + d est supérieur ou égal à 4 et c est un nombre supérieur ou égal à 4.

5. Composition selon la revendication 4, caractérisée par le fait que les copolymères blocs linéaires polysiloxane-polyoxyalkylène ont pour motif récurrent celui de structure :
[- (SiMe₂O)ₓSiMe₂-C₄H₈O-(C₂H₄O)y-(C₃H₆O)_{z}-C₄H₈-]
où x est un nombre compris entre 5 et 15 (bornes incluses), y est un nombre compris entre 15 et 30 (bornes incluses); et z est un nombre compris entre 20 et 40 (bornes incluses).

6. Composition selon l'une quelconque des revendications 1 à 4, caractérisée en ce que le copolymère bloc est choisi parmi le groupe comprenant les composés suivants :
[[(CH₃)₂SiO]₄₁(CH₃)₂SiCH₂CH(CH₃)CH₂-O(C₂H₄O)₁₈-(C₃H₆O)₃₃CH₂CH(CH₃)CH₂]_{16.1}
[[(CH₃)₂SiO]₃₁(CH₃)₂SiCH₂CH(CH₃)CH₂-O(C₂H₄O)₂₀-(C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈-(C₃H₆O)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}

7. Composition selon l'une quelconque des revendications 1 à 6, caractérisée par le fait que l'alcool hydrosoluble est choisi parmi les alcools en C₁-C₄ tel que l'éthanol, l'éthylèneglycol, le propylène glycol, le butylène glycol, l'isopropanol, les éthers de glycol tel que les alkyl(C₁-C₄)éthers de mono di ou tripropylène glycol, mono di ou triéthylène glycol, le dipropylène glycol, le diéthylène glycol et leurs mélanges.

8. Compositions selon l'une quelconque des revendications précédentes, caractérisées par le fait que la silicone insoluble non volatile est choisie parmi les huiles de silicone, les gommes de silicone et les résines de silicone.

9. Compositions selon la revendication 8, caractérisées par le fait que la silicone insoluble non volatile est choisie parmi les polydialkylsiloxanes ou les polyalkylarylsiloxanes, éventuellement modifiés.

10. Compositions selon la revendication 8, caractérisées par le fait que la silicone insoluble non volatile est choisie parmi les gommes de polydialkylsiloxane ou de polyalkylarylsiloxane, éventuellement modifées.

11. Compositions selon l'une quelconque des revendications précédentes caractérisées par le fait que les huiles ou cires fluorées sont choisies parmi les perfluoropolyéthers et les huiles fluorohydrocarbonées.

12. Composition selon l'une quelconque des revendications 1 à 11, caractérisée par le fait que l'agent de conditionnement est présent dans des proportions comprises entre 0,05 et 15% en poids par rapport à l'ensemble de la composition.

13. Composition selon la revendication 12, caractérisée par le fait que l'agent de conditionnement est présent dans des proportions comprises entre 0,1 et 10 % en poids par rapport à l'ensemble de la composition.

14. Composition selon l'une quelconque des revendications précédentes caractérisée par le fait que le copolymère bloc linéaire polysiloxane-polyoxyalkylène est présent dans des proportions comprises entre 0,01 et 15% en poids par rapport à l'ensemble de la composition.

15. Composition selon la revendication 14, caractérisée par le fait que le copolymère bloc linéaire polysiloxane-polyoxyalkylène est présent dans des proportions comprises entre 0,1 et 10 % en poids par rapport à l'ensemble de la composition.

16. Composition selon l'une quelconque des revendications précédentes, caractérisée par le fait que les alcools hydrosolubles sont présents dans des proportions comprises entre 0,5 et 80 % en poids, de préférence de 3 à 40 % en poids et encore plus particulièrement de 5 à 20% en poids, par rapport au poids total de la composition.

17. Utilisation d'une composition telle que définie dans l'une quelconque des revendications 1 à 16, comme, ou pour la fabrication de, compositions cosmétiques pour le maintien de la coiffure, le traitement, le soin ou le lavage de la peau, des cheveux ou de toute autre matière kératinique.

18. Utilisation selon la revendication 17, caractérisée par le fait que lesdites compositions cosmétiques consistent en des lotions de mise en plis, des lotions pour le brushing, des compositions de coiffage ou de fixation, des compositions de shampooing, des compositions à rincer ou non à appliquer avant ou après un shampooing, une coloration ou une décoloration, une permanente ou un défrisage, des compositions de coloration, de décoloration, de permanente ou de défrisage des cheveux.

19. Procédé de traitement cosmétique de la peau ou des fibres kératiniques telles que les cheveux, caractérisé par le fait que l'on applique sur ceux-ci au moins une composition telle que définie dans l'une quelconque des revendications 1 à 16.

20. Utilisation d'un mélange copolymère bloc linéaire polysiloxane-polyoxyalkylène defini selon l'une quelconque des revendications 1 à 6 alcool hydrosoluble comme agent de mise en dispersion d'un agent de conditionnement insoluble choisi parmi les silicones non volatiles et les huiles ou cires fluorées dans une composition cosmétique contenant un tel agent de conditionnement dans un milieu aqueux.

## Claims

1. Cosmetic composition, characterized in that it comprises, in a cosmetically acceptable aqueous medium, at least one insoluble conditioning agent chosen from non-volatile silicones and fluorinated oils or waxes, at least one water-soluble alcohol and at least one polysiloxane-polyoxyalkylene linear block copolymer corresponding to the general formula:
([Y(R₂SiO)ₐR'₂SiYO][(CₙH₂ₙO)_{b}])_{c} (I)
in which:
- R and R', which are identical or different, represent a monovalent hydrocarbon radical which does not contain aliphatic unsaturation,
- n is an integer ranging from 2 to 4,
- a is an integer greater than or equal to 5,
- b is an integer greater than or equal to 4,
- c is an integer greater than or equal to 4,
- Y represents a divalent organic group which is bonded to the adjacent silicon atom via a carbon-silicon bond and to a polyoxyalkylene block via an oxygen atom,
- the mean molecular weight of each siloxane block is between approximately 400 and approximately 10,000, that of each polyoxyalkylene block being between approximately 300 and approximately 10,000,
- the siloxane blocks represent from approximately 10% to approximately 95% by weight of the block copolymer,
- the mean molecular weight of the block copolymer being at least 3000.

2. Composition according to Claim 1, characterized in that R and R' are chosen from the group comprising the methyl, ethyl, propyl, butyl, pentyl, hexyl, octyl, decyl, dodecyl, phenyl, naphthyl, benzyl, phenylethyl, tolyl, xylyl and cyclohexyl radicals.

3. Composition according to either of Claims 1 and 2, characterized in that Y is chosen from the group comprising -R''-, -R''-CO-, -R''-NHCO-, -R''-NH-CO-NH-R'''NHCO- or - R''-OCONH-R'''-NHCO-, where R'' represents an ethylene, propylene or butylene radical and R''' represents a -C₆H₄-, -C₆H₄-C₆H₄-, -C₆H₄-CH₂-C₆H₄- or -C₆H₄-C(CH₃)₂-C₆H₄- group.

4. Composition according to any one of Claims 1 to 3, characterized in that the polysiloxane-polyoxyalkylene linear block copolymers are chosen from those of formula:
[C₄H₈O(CₙH₂ₙO)_{b}(CₘH₂ₘO)_{d}-C₄H₈-SiMe₂O(SiMe₂O)ₚSiMe₂]_{c}
where Me represents methyl, n and m are integers ranging from 2 to 4, p is an integer greater than or equal to 4, b and d are integers greater than or equal to 0, b + d is greater than or equal to 4 and c is a number greater than or equal to 4.

5. Composition according to Claim 4, characterized in that the polysiloxane-polyoxyalkylene linear block copolymers have for a repeat unit that of structure:
[-(SiMe₂O)ₓSiMe₂-C₄H₈O-(C₂H₄O)_{y}-(C₃H₆O)ₓ-C₄H₈-]
where x is a number between 5 and 15 inclusive, y is a number between 15 and 30 inclusive and z is a number between 20 and 40 inclusive.

6. Composition according to any one of Claims 1 to 4, characterized in that the block copolymer is chosen from the group comprising the following compounds:
[[(CH₃)₂SiO]₄₁(CH₃)₂SiCH₂CH(CH₃)CH₂-O(C₂H₄O)₁₈-(C₃H₆O)₃₃CH₂CH(CH₃)CH₂]_{16.1}
[[(CH₃)₂SiO]₃₁(CH₃)₂SiCH₂CH(CH₃)CH₂-O(C₂H₄O)₂₀-(C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈-(C₃H₆O)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}

7. Composition according to any one of Claims 1 to 6, characterized in that the water-soluble alcohol is chosen from C₁-C₄ alcohols, such as ethanol, ethylene glycol, propylene glycol, butylene glycol or isopropanol, glycol ethers, such as the (C₁-C₄)alkyl ethers of mono-, di- or tripropylene glycol or mono-, di- or triethylene glycol, dipropylene glycol, diethylene glycol and their mixtures.

8. Compositions according to any one of the preceding claims, characterized in that the non-volatile insoluble silicone is chosen from silicone oils, silicone gums and silicone resins.

9. Compositions according to Claim 8, characterized in that the non-volatile insoluble silicone is chosen from optionally modified polydialkylsiloxanes or polyalkylarylsiloxanes.

10. Compositions according to Claim 8, characterized in that the non-volatile insoluble silicone is chosen from optionally modified polydialkylsiloxane or polyalkylarylsiloxane gums.

11. Compositions according to any one of the preceding claims, characterized in that the fluorinated oils or waxes are chosen from perfluoropolyethers and fluorohydrocarbon oils.

12. Composition according to any one of Claims 1 to 11, characterized in that the conditioning agent is present in proportions of between 0.05 and 15% by weight with respect to the whole of the composition.

13. Composition according to Claim 12, characterized in that the conditioning agent is present in proportions of between 0.1 and 10% by weight with respect to the whole of the composition.

14. Composition according to any one of the preceding claims, characterized in that the polysiloxane-polyoxyalkylene linear block copolymer is present in proportions of between 0.01 and 15% by weight with respect to the whole of the composition.

15. Composition according to Claim 14, characterized in that the polysiloxane-polyoxyalkylene linear block copolymer is present in proportions of between 0.1 and 10% by weight with respect to the whole of the composition.

16. Composition according to any one of the preceding claims, characterized in that the water-soluble alcohols are present in proportions of between 0.5 and 80% by weight, preferably of 3 to 40% by weight and more particularly still of 5 to 20% by weight, with respect to the total weight of the composition.

17. Use of a composition as defined in any one of Claims 1 to 16 as, or for the manufacture of, cosmetic compositions for retaining the hairstyle or treating, caring for or washing the skin, the hair or any other keratinous material.

18. Use according to Claim 17, characterized in that the said cosmetic compositions comprise hair-setting lotions, blow-drying lotions, styling or setting compositions, shampooing compositions, rinsing or non-rinsing compositions to be applied before or after shampooing, dyeing or bleaching, perming or hair straightening, or dyeing, bleaching, perming or straightening compositions for the hair.

19. Process for the cosmetic treatment of the skin or of keratinous fibres such as hair, characterized in that at least one composition as defined in any one of Claims 1 to 16 is applied to the latter.

20. Use of a polysiloxane-polyoxyalkylene linear block copolymer defined according to any one of Claims 1 to 6/water-soluble alcohol mixture as agent for dispersing an insoluble conditioning agent chosen from non-volatile silicones and fluorinated oils or waxes in a cosmetic composition containing such a conditioning agent in an aqueous medium.

## Patentansprüche

1. Kosmetische Zusammensetzung, dadurch gekennzeichnet, daß sie in einem kosmetisch akzeptablen wässerigen Medium mindestens ein unlösliches Konditioniermittel, das unter den nichtflüchtigen Siliconen und fluorierten Ölen oder Wachsen ausgewählt ist, mindestens einen wasserlöslichen Alkohol und mindestens ein lineares Polysiloxan-Polyoxyalkylen-Blockcopolymer enthält, das der allgemeinen Formel:
([Y(R₂SiO)ₐR'₂SiYO][(CₙH₂ₙO)_{b}])_{c} (I)
entspricht, worin bedeuten:
- R und R', die gleich oder voneinander verschieden sind, eine einwertige Kohlenwasserstoffgruppe, die keine aliphatischen Doppelbindungen enthält,
- n eine ganze Zahl von 2 bis 4,
- a eine ganze Zahl von mindestens 5,
- b eine ganze Zahl von mindestens 4,
- c eine ganze Zahl von mindestens 4,
- Y eine zweiwertige organische Gruppe, die an das angrenzende Siliciumatom über eine Kohlenstoff-Silicium-Bindung und an einen Polyoxyalkylenblock über ein Sauerstoffatom gebunden ist,
- das mittlere Molekulargewicht jedes Siloxanblocks liegt im Bereich von etwa 400 bis etwa 10000, das mittlere Molekulargewicht jedes Polyoxyalkylenblocks im Bereich von etwa 300 bis etwa 10000,
- die Siloxanblöcke machen etwa 10 % bis etwa 95 % des Gewichts des Blockcopolymers aus,
- das mittlere Molekulargewicht des Blockcopolymers beträgt mindestens 3000.

2. Zusammensetzung nach Anspruch 1, dadurch gekennzeichnet, daß R und R' unter den Gruppen Methyl, Ethyl, Propyl, Butyl, Pentyl, Hexyl, Octyl, Decyl, Dodecyl, Phenyl, Naphthyl, Benzyl, Phenylethyl, Tolyl, Xylyl und Cyclohexyl ausgewählt sind.

3. Zusammensetzung nach einem der Ansprüche 1 oder 2, dadurch gekennzeichnet, daß Y unter -R''-, -R''-CO-, -R''-NHCO-, -R''NH-CONH-R'''-NHCO-, -R''-OCONH-R'''-NHCO-ausgewählt ist, worin R'' eine Ethylen-, Propylen- oder Butylengruppe, und R'''eine Gruppe -C₆H₄-, -C₆H₄-C₆H₄-, -C₆H₄-CH₂-C₆H₄-, -C₆H₄-C(CH₃)₂-C₆H₄- bedeuten.

4. Zusammensetzung nach einem der Ansprüche 1 bis 3, dadurch gekennzeichnet, daß die lineare Polysiloxan-Polyoxyalkylen-Blockcopolymere unter den Blockcopolymeren der Formel:
[C₄H₈O(CₙH₂ₙO)_{b}(CₘH₂ₘO)_{d}-C₄H₈-SiMe₂O(SiMe₂O)ₚSiMe₂]_{c}
ausgewählt sind, worin bedeuten: Me Methyl, n und m ganze Zahlen im Bereich von 2 bis 4, p eine ganze Zahl von mindestens 4, b und d Null oder eine ganze Zahl über Null, b+d mindestens 4 und c eine ganze Zahl von mindestens 4.

5. Zusammensetzung nach Anspruch 4, dadurch gekennzeichnet, daß die linearen Polysiloxan-Polyoxyalkylen-Blockcopolymere wiederkehrende Einheiten der folgenden Struktur aufweisen:
[-(SiMe₂O)ₓSiMe₂-C₄H₈O-(C₂H₄O)_{y}-(C₃H₆O)_{z}-C₄H₈-],
wobei x eine ganze Zahl im Bereich von 5 bis 15 (Grenzen eingeschlossen), y eine ganze Zahl von 15 bis 30 (Grenzen eingeschlossen), und z eine ganze Zahl im Bereich von 20 bis 40 (Grenzen eingeschlossen) bedeuten.

6. Zusammensetzung nach einem der Ansprüche 1 bis 4, dadurch gekennzeichnet, daß das Blockcopolymer unter den folgenden Verbindungen ausgewählt ist:
[[(CH₃)₂SiO]₄₁(CH₃)₂SiCH₂CH(CH₃)CH₂-O(C₂H₄O)₁₈-(C₃H₆O)₃₃CH₂CH(CH₃)CH₂]_{16.1}
[[(CH₃)₂SiO]₃₁(CH₃)₂SiCH₂CH(CH₃)CH₂-O(C₂H₄O)₂₀-(C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈-(C₃H₆O)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}.

7. Zusammensetzung nach einem der Ansprüche 1 bis 6, dadurch gekennzeichnet, daß der wasserlösliche Alkohol unter den C₁₋₄-Alkoholen, wie Ethanol, Ethylenglykol, Propylenglykol, Butylenglykol und Isopropanol, den Glykolethern, wie den C₁₋₄-Alkylethern von Mono-, Di- oder Tripropylenglykol, Mono-, Di- oder Triethylenglykol, Dipropylenglykol, Diethylenglykol und deren Gemischen ausgewählt ist.

8. Zusammensetzung nach einem der vorhergehenden Ansprüche, dadurch gekennzeichnet, daß das nichtflüchtige unlösliche Silicon unter den Siliconölen, Silicongummis und Siliconharzen ausgewählt ist.

9. Zusammensetzung nach Anspruch 8, dadurch gekennzeichnet, daß das nichtflüchtige unlösliche Silicon unter den Polydialkylsiloxanen oder den Polyalkylarylsiloxanen, die gegebenenfalls modifiziert sind, ausgewählt ist.

10. Zusammensetzung nach Anspruch 8, dadurch gekennzeichnet, daß das unlösliche nichtflüchtige Silicon unter den Polydialkylsiloxangummis oder Polyalkylarylsiloxangummis, die gegebenenfalls modifiziert sind, ausgewählt ist.

11. Zusammensetzung nach einem der vorhergehenden Ansprüche, dadurch gekennzeichnet, daß die fluorierten Öle oder Wachse unter den Perfluorpolyethern und den Fluorkohlenwasserstoffölen ausgewählt sind.

12. Zusammensetzung nach einem der Ansprüche 1 bis 11, dadurch gekennzeichnet, daß das Konditioniermittel in Anteilen von 0,05 bis 15 Gew.-%, bezogen auf die gesamte Zusammensetzung, vorliegen.

13. Zusammensetzung nach Anspruch 12, dadurch gekennzeichnet, daß das Konditioniermittel in Mengenanteilen im Bereich von 0,1 bis 10 Gew.-%, bezogen auf die gesamte Zusammensetzung, vorliegt.

14. Zusammensetzung nach einem der vorhergehenden Ansprüche, dadurch gekennzeichnet, daß das lineare Polysiloxan-Polyoxyalkylen-Blockcopolymer in Anteilen von 0,01 bis 15 Gew.-%, bezogen auf die gesamte Zusammensetzung, vorliegt.

15. Zusammensetzung nach Anspruch 14, dadurch gekennzeichnet, daß das lineare Polysiloxan-Polyoxyalkylen-Blockcopolymer in Anteilen von 0,1 bis 10 Gew.-%, bezogen auf die gesamte Zusammensetzung, vorliegt.

16. Zusammensetzung nach einem der vorhergehenden Ansprüche, dadurch gekennzeichnet, daß die wasserlöslichen Alkohole in Anteilen von 0,5 bis 80 Gew.-% und vorzugsweise von 3 bis 40 Gew.-% und noch bevorzugter von 5 bis 20 Gew.-%, bezogen auf die gesamte Zusammensetzung, vorliegen.

17. Verwendung einer Zusammensetzung nach einem der Ansprüche 1 bis 16 als kosmetische Zusammensetzung für die Festigung der Frisur, die Behandlung, Pflege oder das Waschen der Haut, der Haare oder beliebiger anderer Keratinmaterialien oder zu deren Herstellung.

18. Verwendung nach Anspruch 17, dadurch gekennzeichnet, daß die kosmetischen Zusammensetzungen Lotionen für Wasserwellen, Brushinglotionen, Zusammensetzungen zum Frisieren oder Fixieren, Haarwaschmittelzusammensetzungen, Zusammensetzungen, die ausgespült werden oder im Haar verbleiben und die vor oder nach Haarwäschen, Färbungen oder Entfärbungen, permanenten Verformungen oder Entkräuselungen angewandt werden, Zusammensetzungen zum Färben, Entfärben, für permanente Verformungen oder zum Entkräuseln von Haaren sind.

19. Verfahren zur kosmetischen Behandlung der Haut oder von Keratinfasern, wie dem Haar, dadurch gekennzeichnet, daß auf diese mindestens eine Zusammensetzung nach einem der Ansprüche 1 bis 16 aufgetragen wird.

20. Verwendung eines Gemisches eines linearen Polysiloxan-Polyoxyalkylen-Blockcopolymers und eines wasserlöslichen Alkohols als Mittel zum Dispergieren eines unlöslichen Konditioniermittels, das unter den nichtflüchtigen Siliconen und den fluorierten Ölen oder Wachsen ausgewählt ist, in einer kosmetischen Zusammensetzung, die ein solches Konditioniermittel in einem wässerigen Medium enthält.
